# EUROPÄISCHE PATENTSCHRIFT

(11) **EP 1 443 979 B1**
(45) Veröffentlichungstag und Bekanntmachung des Hinweises auf die Patenterteilung: **06.04.2005**
(21) Anmeldenummer: 02803016.1
(22) Anmeldetag: 13.11.2002
(51) Int. Cl.: A61L 2/26, A61B 19/02, A61J 1/00

(54) **STERILBEHÄLTER**
STERILE CONTAINER
RECIPIENT STERILE

(30) Priorität: 15.11.2001 DE 10156937
(43) Veröffentlichungstag der Anmeldung: 11.08.2004
(73) Patentinhaber: Aesculap AG & Co. KG, 78532 Tuttlingen (DE)
(72) Erfinder: GLEICHAUF, Wilhelm, 78532 Tuttlingen-Möhringen (DE); JAKAB, Mariana, 78532 Tuttlingen (DE); OERTMANN, Friedrich-Wilhelm, 78532 Tuttlingen (DE); RENNER, Torsten, 78532 Tuttlingen (DE); SCHUSTER, Stefan, 78048 Villingen-Schwenningen (DE); SCHWANKE, Wolfgang, 78604 Rietheim-Weilheim (DE)
(74) Vertreter: HOEGER, STELLRECHT & PARTNER Patentanwälte
(86) Internationale Anmeldenummer: PCT/EP2002/012670
(87) Internationale Veröffentlichungsnummer: WO 2003/041748

(56) Entgegenhaltungen:
- WO-A-01/08583
- DE-A- 3 407 112
- US-A- 4 728 504
- US-A- 5 176 884
- US-A- 5 183 643
- US-A- 5 324 489

## Beschreibung

Die Erfindung betrifft einen Sterilbehälter, insbesondere zur Aufnahme und sterilen Aufbewahrung von chirurgischem Besteck oder Material, umfassend einen durch einen Behälterboden und Behälterwände gebildeten Aufnahmeraum, einen Deckel zum Verschließen des Aufnahmeraums und eine von einem Sterilfilter verschließbare Gasaustauschöffnung, wobei eine das Sterilfilter, einen Träger und ein Halteelement umfassende Filtereinheit vorgesehen ist, wobei das Sterilfilter zwischen dem Träger und dem Halteelement gehalten ist und wobei die Filtereinheit am Deckel und/oder am Behälterboden gelagert ist.

Derartige Sterilbehälter werden beispielsweise mit chirurgischem Besteck oder Material beschickt und in einem Sterilisator sterilisiert. Ein Sterilfilter ist erforderlich, um das Eindringen von Keimen in den Behälter zu verhindern. Das Sterilfilter muß von Zeit zu Zeit ausgetauscht werden, was bei bisher bekannten Sterilbehältern nur auf umständliche Weise möglich ist.

Ein Beispiel für einen Sterilbehälter gemäß dem Oberbegriff des Anspruchs 1 ist aus der US 5,324,489 bekannt.

Daher ist es Aufgabe der vorliegenden Erfindung, einen Sterilbehälter der eingangs beschriebenen Art so zu verbessern, daß das Sterilfilter einfach ausgetauscht werden kann.

Diese Aufgabe wird bei einem Sterilbehälter der eingangs beschriebenen Art erfindungsgemäß dadurch gelöst, daß die Filtereinheit in Form einer einteiligen Filterkassette ausgebildet ist und daß der Träger, der Sterilfilter und das Halteelement untrennbar miteinander verbunden sind.

Zum Austauschen des Sterilfilters genügt es, die komplette Filtereinheit vom Deckel oder vom Behälterboden zu lösen und auszutauschen. Das Sterilfilter muß dabei nicht mehr selbst gegriffen werden, was einerseits eine Beschädigung'desselben vermeiden hilft, andererseits eine Beschmutzung verhindert. Die Filtereinheit kann also als Ganzes ausgetauscht werden, wodurch ein schneller Filtertausch möglich wird. Gleichzeitig ist das Sterilfilter dauerhaft in der Filterkassette geschützt lagerbar. Ferner kann schon bei der Herstellung der Filterkassette deren Dichtheit geprüft werden. Diese ist bei einer manuell zusammengesetzten Filtereinheit nicht immer gewährleistet.

Um das Sterilfilter sicher zu halten, ist vorteilhafterweise vorgesehen, daß der Träger mindestens eine Trägerauflage zum Lagern des Sterilfilters und mindestens eine an die Trägerauflage angrenzende Trägerdurchbrechung aufweist. Während die Trägerauflage einerseits das Sterilfilter der Filtereinheit stabilisiert, ermöglicht die Trägerdurchbrechung einen ausreichenden Gasaustausch durch das Sterilfilter hindurch.

Um das Sterilfilter vor einer mechanischen Beanspruchung, insbesondere einer Verletzung durch im oder am Sterilbehälter angreifende Instrumente und Werkzeuge zu schützen, ist vorzugsweise mindestens eine die mindestens eine Trägerdurchbrechung beabstandet bedeckende Trägerdurchbrechungsabdeckung vorgesehen.

Von Vorteil kann es sein, wenn die Filtereinheit bewegbar gelagert ist, wenn die Filtereinheit in einer Schließstellung einen Strömungspfad schließt und in einer Durchlaßstellung den Strömungspfad öffnet, so daß ein Gasaustausch in der Schließstellung nur durch das Sterilfilter und in der Durchlaßstellung durch das Sterilfilter und/oder durch den Strömungskanal ermöglicht wird. Durch die bewegbare Lagerung kann ein Gasaustausch zusätzlich gesteuert und/oder geregelt werden. Insbesondere dann, wenn ein größerer Gasaustausch erforderlich ist, kann die Filtereinheit in die Durchlaßstellung überführt und ein zusätzlicher Strömungspfad geöffnet werden.

Um eine keimfreie Aufbewahrung der im Sterilbehälter gelagerten Gegenstände zu ermöglichen, ist die Filtereinheit in der Schließstellung am Sterilbehälter unter Vorspannung gehalten.

Günstig ist es, wenn ein Dichtelement zur gasdichten Lagerung der Filtereinheit vorgesehen ist. Mit diesem kann die Filtereinheit am Sterilbehälter vollständig abgedichtet werden.

Besonders günstig ist es, wenn das Dichtelement einen am Halteelement gelagerten Dichtring umfaßt. Diese sind besonders einfach herzustellen und auf einfache Weise am Halteelement oder einem sonstigen Element der Filtereinheit anzubringen.

Gemäß einer bevorzugten Ausführungsform der Erfindung ist es von Vorteil, wenn ein Überdruckventil vorgesehen ist, wenn das Überdruckventil so angeordnet ist, daß es in einer Grundstellung eine Schließstellung einnimmt und wenn es eine Durchlaßstellung einnimmt, wenn ein Druck in einer Umgebung des Sterilbehälters einen Druck im Sterilbehälter um eine vorgegebene Druckdifferenz übersteigt. So läßt sich eine Beschädigung des Sterilbehälters vermeiden, denn bevor der Sterilbehälter aufgrund eines besonders großen Drucks in der Umgebung oder eines entsprechend hohen Druckgradienten verformt werden kann, öffnet das Überdruckventil und ermöglicht das Einströmen von Gas in den Behälter.

Um die Anzahl der notwendigen Bauelemente zu verringern, ist es von Vorteil, wenn die Filtereinheit das Überdruckventil bildet. Die Filtereinheit erfüllt somit zwei Funktionen, einerseits dient sie dem Zurückhalten von Keimen, andererseits als Überdruckventil.

Besonders günstig ist es, wenn die Filtereinheit auf einer Innenseite des Deckels gelagert ist. Dies schafft zusätzlichen Raum im Behälter. Außerdem kann eine Filtereinheit auch durch Austausch eines kompletten Deckels getauscht werden. Die Lagerung auf der Innenseite des Deckels bietet einen zusätzlichen Schutz für die Filtereinheit gegen mechanische Belastung oder Beanspruchung.

Um die Filtereinheit zusätzlich gegen mechanische Beanspruchung zu schützen, kann es von Vorteil sein, ein die Filtereinheit beabstandet bedeckendes Schutzelement vorzusehen.

Vorteilhaft ist es, wenn das Schutzelement auf einer Außenseite des Deckels angeordnet ist. Dadurch ist das Sterilfilter sicher zwischen dem Schutzelement und dem Träger gelagert.

Um eine einfache Montage zu erreichen oder auf einfache Weise auf die Filtereinheit zuzugreifen, kann vorgesehen sein, daß das Schutzelement mittels einer Schnapp- oder Rastverbindung mit dem Deckel verbindbar ist.

Zur Vermeidung weiterer Öffnungen am Deckel ist es vorteilhaft, wenn das Schutzelement mit einem Innenrand der Gasaustauschöffnung verrastbar ist. Die Gasaustauschöffnung ist in jedem Fall erforderlich, um einen Gasaustausch mit dem Innenraum des Sterilbehälters zu ermöglichen.

Eine besonders einfache Rastverbindung ergibt sich, wenn das Schutzelement in Richtung auf den Sterilbehälter abstehende, mit einem Rastvorsprung versehene elastische Federarme umfaßt. Außer den Federarmen sind sonst keine beweglichen Teile zur Fixierung des Schutzelements am Sterilbehälter erforderlich.

Vorteilhafter Weise kann zwischen dem Schutzelement und dem Deckel mindestens eine mit der Gasaustauschöffnung in Fluidverbindung stehende Gasdurchtrittsöffnung vorgesehen sein, die so angeordnet ist, daß eine Gasströmung in einer im wesentlichen quer zur Durchlaßrichtung des Sterilfilters verlaufenden Strömungsrichtung ermöglicht wird. Über die Gasdurchtrittsöffnung läßt sich ein Gasaustausch von der Umgebung des Sterilbehälters zur Gasaustauschöffnung hin ermöglichen, was insbesondere dann notwendig ist, wenn das Schutzelement auf dem Deckel angeordnet ist.

Um ein Eindringen von Flüssigkeit in die Filtereinheit zu verhindern, ist es günstig, wenn eine auf der Außenseite des Deckels angeordnete und von der Gasaustauschöffnung weg weisende, nach außen relativ zu einer Deckelebene abfallende Einströmkante vorgesehen ist. Gelangt Flüssigkeit auf den Deckel, so kann sie aufgrund der Neigung der Einströmkante von der Gasaustauschöffnung weg fließen und somit nicht auf die Filtereinheit gelangen.

Vorzugsweise weist der Deckel mindestens ein Abstandselement auf zum Stapeln eines weiteren Sterilbehälters auf dem Sterilbehälter, so daß ein Gasaustausch durch die Gasaustauschöffnung bei gestapelten Sterilbehältern möglich ist. Einerseits wird somit ein Gasaustausch ermöglicht, andererseits auch die Gasaustauschöffnung und der diese bedeckende Sterilfilter geschützt gegen darauf abgelegte weitere Sterilbehälter.

Gemäß einer bevorzugten Ausführungsform der Erfindung kann vorgesehen sein, daß das Sterilfilter ein Dauerfilter ist, insbesondere ein aus Polytetrafluorethylen (PTFE) hergestelltes. Damit lassen sich Wartungsintervalle des Sterilbehälters verlängern. Ein Austausch der Filtereinheit ist nur noch selten erforderlich.

Besonders günstig und einfach in der Herstellung wird der Sterilbehälter dann, wenn der Deckel aus einem Kunststoff hergestellt ist, insbesondere aus Polyetheretherketon (PEEK) oder Polyphenylensulfon (PPSU).

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Gesamtansicht eines Sterilbehälters;
- Figur 2:: eine ausschnittsweise Draufsicht auf einen Sterilbehälter;
- Figur 3:: eine ausschnittsweise Explosionsdarstellung eines Deckels des Sterilbehälters;
- Figur 4:: eine teilweise durchbrochene Querschnittsansicht durch einen Ausschnitt eines Deckels;
- Figur 5:: eine ausschnittsweise, teilweise durchbrochene Seitenansicht eines Deckels;
- Figur 6:: eine vergrößerte perspektivische Teilansicht eines Dekkels;
- Figur 7:: eine Draufsicht auf die Unterseite eines halben Deckels;
- Figur 8:: eine ausschnittsweise Querschnittsansicht einer gasdicht am Deckel gelagerten Filterkassette; und
- Figur 9:: eine ausschnittsweise Querschnittsansicht ähnlich Figur 8 mit einer abgehobenen Filterkassette.

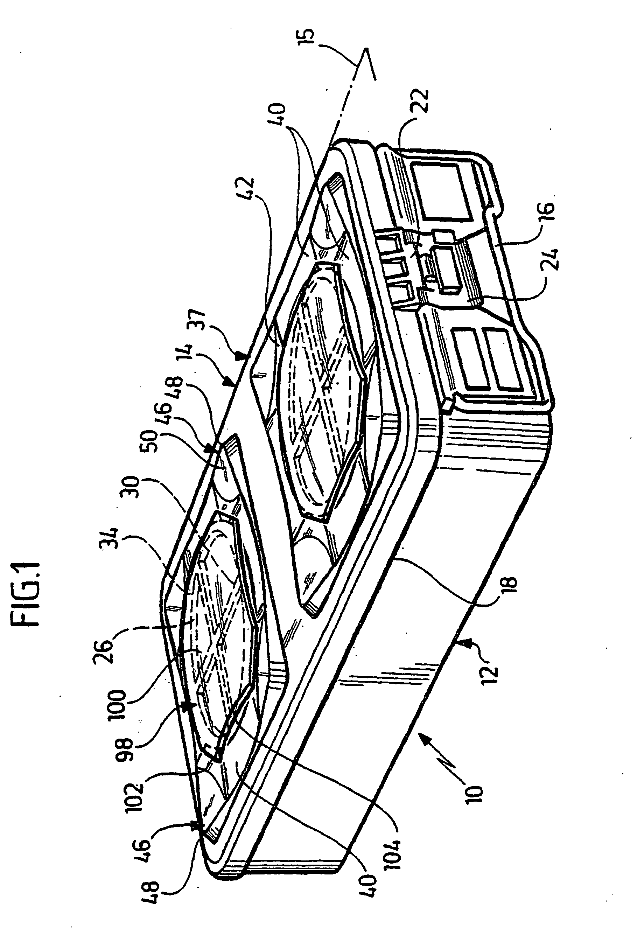

In Figur 1 ist ein insgesamt mit dem Bezugszeichen 10 versehener Sterilbehälter dargestellt, der einen im wesentlichen wannenartigen, quaderförmigen Behälter 12 zum Aufnehmen beispielsweise von chirurgischem Besteck oder Material und einen Deckel 14 umfaßt. An den Stirnflächen des Behälters 12 sind schwenkbar gelagerte Tragegriffe 16 angeordnet.

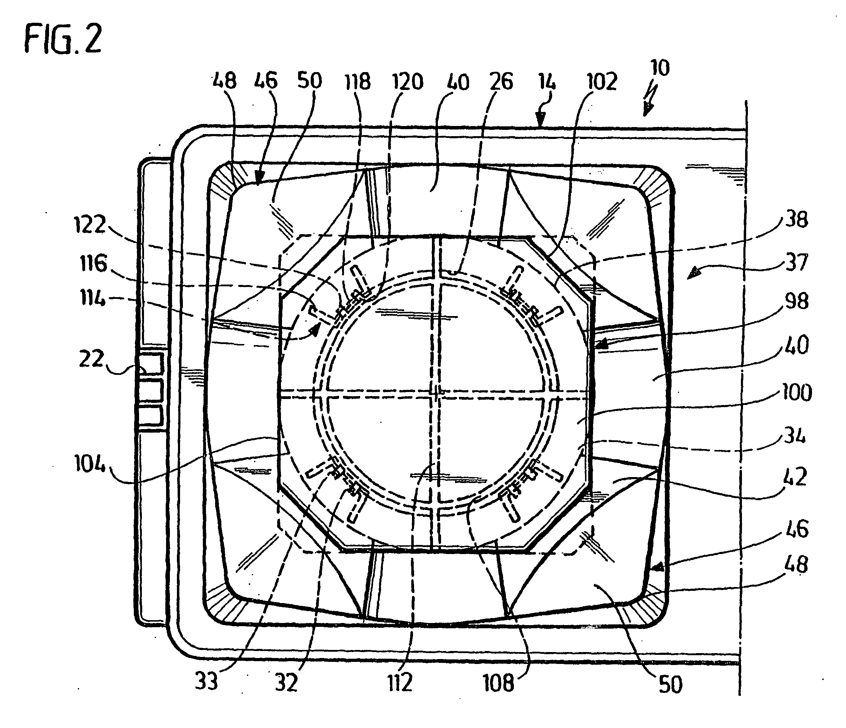

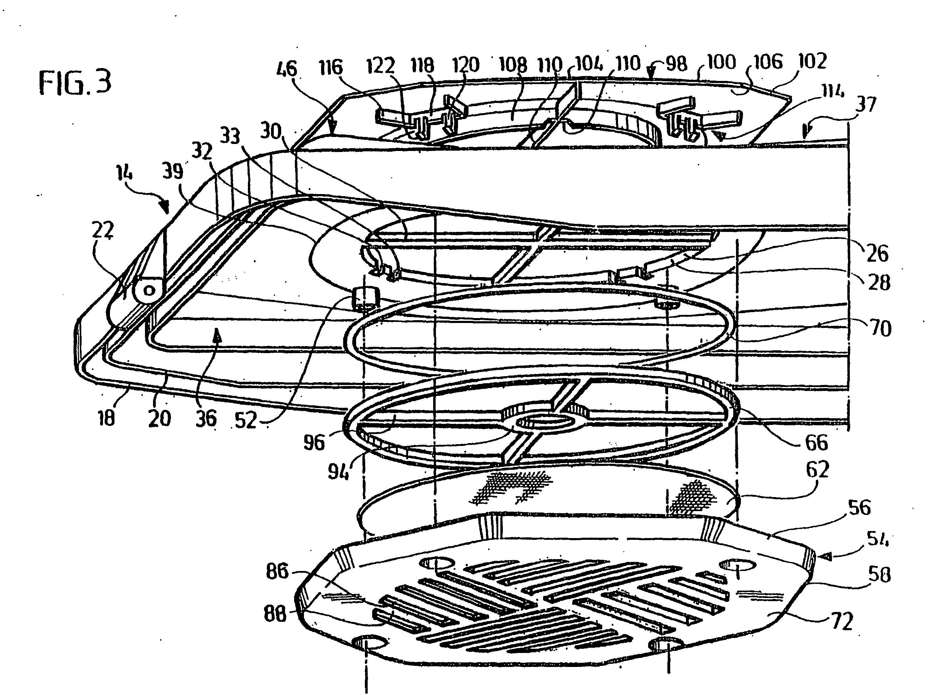

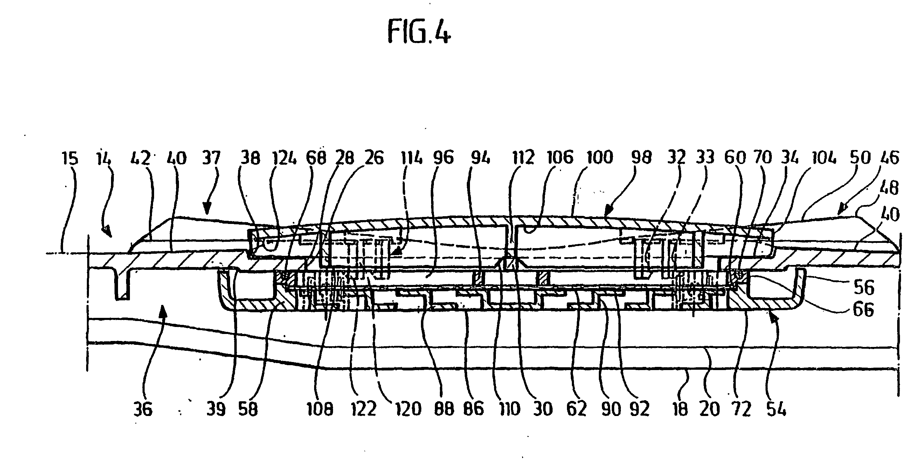

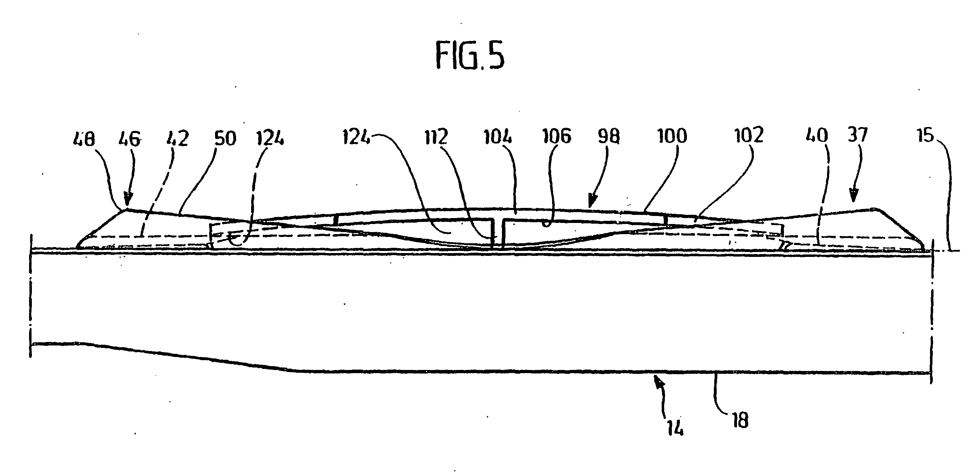

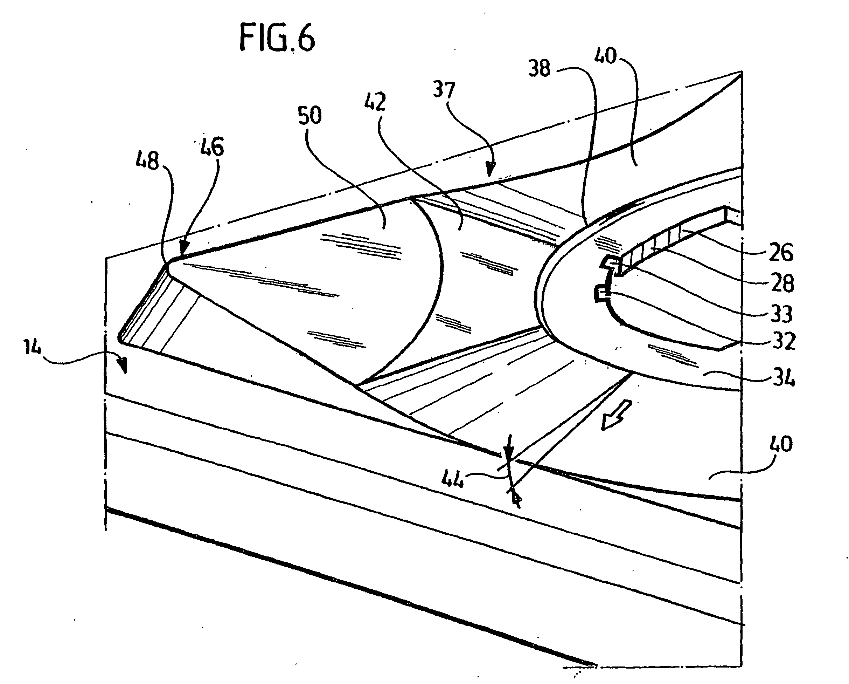

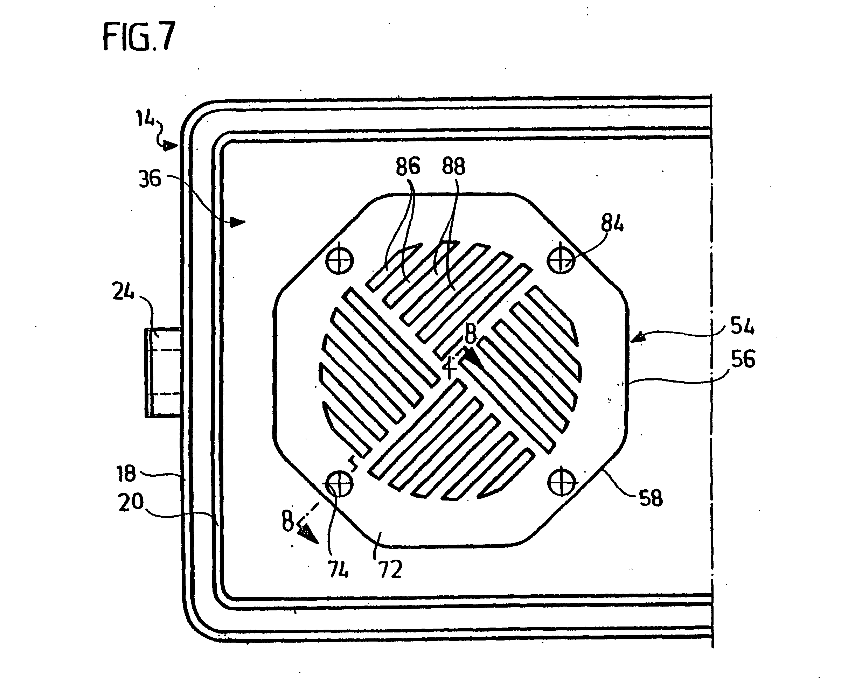

Der Aufbau des Deckels 14 wird anhand der Figuren 2 bis 9 näher erläutert.

Der Deckel 14 ist mit einem umlaufenden, von einer Deckelebene 15 senkrecht abstehenden Rand 18 versehen, so daß der Deckel 14 den Behälter 12 vollständig bedeckt und teilweise umgreift. Parallel zum Rand 18 verläuft beabstandet ein Dichtrand 20, zwischen welchem und dem Rand 18 eine nicht dargestellte Dichtung eingesetzt werden kann. Der Dichtrand 20 befindet sich bei aufgesetztem Deckel 14 innerhalb des Behälters 12. Mit der Dichtung läßt sich eine gasdichte Verbindung zwischen dem Deckel 14 und dem Behälter 12 herstellen.

An seinen schmalen Stirnseiten weist der Deckel 14 jeweils einen Lagerbock 22 auf, an dem eine federnde Verschlußklappe 24 um eine Schwenkachse verschwenkbar ist, die parallel zu einer Schwenkachse des Tragegriffs 16 verläuft, wenn der Deckel 14 auf dem Behälter 12 sitzt.

Der Deckel 14 weist zwei symmetrisch angeordnete kreisförmige Öffnungen 26 auf, die einen parallel zur Deckelebene 15 weisenden Öffnungsrand 28 aufweisen, an dem vier, senkrecht aufeinander zulaufende, sich im Zentrum der Öffnung 26 treffende Stege 30 zur Versteifung des Deckels symmetrisch angeordnet sind. Jeweils zwischen zwei am Öffnungsrand 28 angeordneten Stegen 30 sind symmetrisch zwei Rücksprünge 32 und 33 in den Öffnungsrand 28 eingeschnitten.

Die Öffnung 26 wird von einer Ringfläche 34 umgeben, die parallel zur Deckelebene 15 verläuft, jedoch in Richtung auf eine Deckelunterseite 36 zurückgesetzt ist. Dadurch wird auf einer Deckeloberseite 37 eine innenliegende Ringkante 38, auf der Deckelunterseite 36 eine außenliegende Ringkante 39 gebildet. An die Ringkante 38 grenzen abwechselnd jeweils vier Einströmkanten 40 und vier Auflageflächen 42 an. Die Einströmkanten 40 sind von der Deckeloberseite 37 weg weisend konkav gekrümmt und nach außen geneigt, so daß eine schiefe Ebene mit einem Neigungswinkel 44 zwischen der Einströmkante 40 und der Deckelebene 15 von etwa 2° gebildet wird.

Die Auflageflächen 42 verlaufen parallel zur Deckelebene 15 und weisen in Aufsicht in etwa die Form eines Trapezes auf, jedoch jeweils mit konkav gekrümmten Basiskanten. An die längere der beiden Basislinien der Auflagefläche 42 schließt sich eine dreiseitige Pyramide 46 an, die eine im wesentlichen die Form eines gleichschenkligen rechtwinkligen Dreiecks aufweisende Grundfläche hat. Eine Spitze 48 der Pyramide liegt nahe über den sich rechtwinklig schneidenden Grundseiten der Grundfläche, etwas in Richtung auf die Öffnung 26 hin versetzt.

Dadurch wird eine in etwa die Form eines gleichschenkligen, rechtwinkligen Dreiecks aufweisende Ablauffläche 50 gebildet, die in Richtung auf die Auflagefläche 42 hin geneigt ist. Die Auflagefläche 42 ist parallel zur Deckelebene 15 von der Deckeloberseite 37 weg versetzt.

Insgesamt entsteht somit ein in etwa quadratischer Aufbau auf dem Deckel 14, der gebildet wird durch vier Pyramiden 46, die jeweils in den Ecken des Quadrates sitzen, wobei die Seitenflächen des Quadrats parallel zu Seiten des Deckels 14 verlaufen. Die Einströmkanten 40 sind somit senkrecht zu den Seiten des Deckels 14 hin geneigt. Der Aufbau weist insgesamt zwei parallel zu den Seiten des Deckels 14 verlaufende Symmetrieebenen auf. Die Einströmkante 40 reicht auf ihrer von der Öffnung 26 weg weisenden Seite bis auf das Niveau der Deckelebene 15 herab. In einer seitlichen Ansicht des Deckels ergibt sich somit in etwa eine konkave, von der Deckeloberseite 37 weg weisende Form von der Spitze 48 der Pyramide 46 in einer Ecke des quadratischen Aufbaus bis zur Spitze 48 der Pyramide 46 in einer benachbarten Ecke des Aufbaus.

Benachbart zu den Rücksprüngen 32 und 33 sind insgesamt vier hohlzylindrische, von der Deckelunterseite 36 abstehende, offene Hülsen 52 angeordnet. An diesen ist eine Filterkassette 54 federnd gelagert. Sie umfaßt einen in etwa achteckigen, mit einem Trägerrand 56 versehenen Filterhalter 58 auf, der eine ringförmige Filteraufnahme 60 zum Aufnehmen eines kreisrunden Dauerfilters 62 aufweist. Ein ringförmiger, im Durchmesser größer als die Filteraufnahme 60 ausgestalteter Rücksprung 64 dient zur Aufnahme eines Halteringes 66, der im Querschnitt ein L-förmiges Profil aufweist, dessen einer Schenkel den Dauerfilter 62 in die Filteraufnahme 60 drückt und dessen anderer Schenkel an dem Rücksprung 64 anliegt. Der Haltering 66 ist mit einer ringförmigen, im Querschnitt etwas mehr als die Hälfte eines Kreises aufweisenden Ringnut zur Aufnahme eines Dichtringes 70 versehen.

Von einem Filterhalterboden 72 stehen korrespondierend zu den am Deckel 14 angeordneten Hülsen 52 vier Lagerhülsen 74 in Richtung auf den Deckel ab, die vom Filterhalterboden 72 her offen sind und deren andere verschlossene Stirnfläche 76 mit einer zentralen, im Durchmesser zur Hülse 52 korrespondierenden Durchtrittsöffnung 78 versehen ist. Zur Lagerung der Filterkassette 54 werden die vier Lagerhülsen 74 über die Hülsen 52 geschoben, jeweils eine Spiralfeder 80 über die Hülsen 52 gesteckt und anschließend die Lagerhülsen 74 vom Filterhalterboden 72 mit einem Stopfen 82 verschlossen, der einen scheibenförmigen, im Außendurchmesser an den Innendurchmesser der Lagerhülsen 74 angepaßten Kopf 84 aufweist. Indem sich die Spiralfeder 80 am Kopf 84 des Stopfens 82 abstützt, drückt sie die Stirnfläche 76 gegen die Deckelunterseite 36.

Der Filterhalterboden 72 ist insgesamt mit zwanzig unterschiedlich langen streifenförmigen Schlitzen 86 versehen, wobei jeweils fünf Schlitze parallel zueinander verlaufen und in etwa die Fläche eines Viertelkreises bedecken. Schlitze 86 benachbarter Viertelkreise stehen jeweils senkrecht zueinander. Zwischen jeweils zwei Schlitzen 86 wird jeweils ein Schutzsteg 88 gebildet. An jedem Schutzsteg 88 ist eine vom Filterhalterboden 72 weg weisende Leiste 90 angeordnet, an der wiederum parallel zum Filterhalterboden 72 verlaufend ein vom zugehörigen Schutzsteg 88 weg weisender Trägersteg 92 absteht, der jeweils einen Schlitz 86 vollständig überdeckt. Alle Trägerstege 92 bilden eine gemeinsame Ebene, auf der der Dauerfilter 62 aufliegt. Insgesamt bilden die Filteraufnahme 60 sowie die Trägerstege 92 eine Trägerauflage zum Lagern des Dauerfilters 62.

Der Filterhalter 58, der Dauerfilter 62 und der Haltering 66 sind fest miteinander verbunden, beispielsweise geklebt oder geschweißt. Im montierten Zustand drücken die Spiralfedern 88 die Filterkassette 54 gegen die Deckelunterseite 36, wobei mittels des Dichtringes 70 die Filterkassette 54 an der Deckelunterseite 36 abgedichtet wird.

Der Haltering 66 weist einen äußeren und einen konzentrisch dazu verlaufenden inneren Ring 94 auf, von dem radial und symmetrisch angeordnet vier Halteringstege 96 den Haltering 66 stabilisieren.

Durch die versetzte Anordnung von Trägersteg 92 und Schutzsteg 88 ist der auf den Trägerstegen 92 gelagerte Dauerfilter 62 gegen eine direkte mechanische Verletzung geschützt. Auf seiner in Richtung der Öffnung 26 hin zugewandten Seite ist der Dauerfilter 62 jedoch praktisch ungeschützt. Als Schutzelement ist hierfür ein spiegelsymmetrisch ausgebildeter Schutzdeckel 98 vorgesehen, der eine von der Deckeloberseite 37 weg konvex gekrümmte Deckelfläche 100 aufweist, die in Draufsicht die Form eines ungleichseitigen Achtecks mit kurzen Seiten 102 und langen Seiten 104 hat.

Von einer Schutzdeckelunterseite 106 steht eine Ringleiste 108 ab, die vier jeweils paarweise diametral angeordnete Aussparungen 110 umfaßt. Diese sind derart geformt, daß sie die Stege 30 übergreifen können und der Schutzdeckel 98 auf den Stegen 30 aufliegt. Als zusätzliche Abstandshalter sind zwei sich kreuzende Abstandsleisten 112 an der Schutzdeckelunterseite 106 angeordnet, die im Bereich der Aussparungen 110 über die Ringleiste 108 nach außen bis zu den langen Seiten 104 vorstehen. Diese Leisten liegen bei aufgesetztem Schutzdeckel 98 auf der Ringfläche 34 auf.

Zum Befestigen des Schutzdeckels 98 sind vier Rastverbinder 114 vorgesehen, die jeweils aus einem aus drei rechtwinklig zueinander angeordneten, einen U-förmigen Rahmen 116 bildenden Schenkeln bestehen, von denen zwei radial von der Ringleiste 108 nach außen abstehen. Sie bilden, wie freie Enden der Abstandsleisten 112, auf der Ringfläche 34 aufliegende Abstandshalter. Von einem Quersteg 118 des Rahmens 116 stehen parallel zwei Rastarme 120 senkrecht von der Schutzdeckelunterseite 106 ab, die an ihren freien Enden mit einer rechtwinklig abstehenden Rastnase 122 versehen sind. Die vier Rastverbinder 114 sind paarweise diametral gegenüberliegend am Schutzdeckel 98 angeordnet.

Zum Befestigen des Schutzdeckels 98 wird dieser senkrecht in Richtung auf die Deckeloberseite 37 hin bewegt und so orientiert, daß die Aussparungen 110 jeweils auf einen Steg 30 hin weisen und die Rastarme 120 jeweils in einen der beiden Rücksprünge 32 und 33 eingleiten. Sobald die Aussparungen 110 auf den Stegen 30, die Abschlußleisten 112 und die Rahmen 116 auf der Ringfläche 34 aufliegen, verrasten die Rastarme 120 mit dem Öffnungsrand 28, indem die Rastnasen 122 den Öffnungsrand 28 hintergreifen.

Die Größe des Schutzdeckels 98 ist so gewählt, daß im eingesetzten Zustand die Ringfläche 34 vollständig überdeckt ist. Die kurzen Seiten 102 liegen dann auf den Auflageflächen 42 auf, die langen Seiten 104 verlaufen parallel zu Seitenkanten des Deckels 14 und überdecken jeweils teilweise die Einströmkanten 40. Bei eingesetztem Schutzdeckel 98 ist der in der Filterkassette 54 gehaltene Dauerfilter 62 auch von seiner anderen Seite her vollständig gegen eine mechanische Zerstörung geschützt.

Bei einer alternativen, in Figur 2 strichpunktiert angedeuteten Variante eines Deckels sind die langen Seiten 104 des Schutzdeckels 98 verlängert. Die Pyramide 46 weist dann in ihrer Ablauffläche 50 eine zusätzliche Ausnehmung auf, in der die kurze Seite 102 des Schutzdeckels 98 eingefügt ist. Auf diese Weise entsteht dann ein nahtloser Übergang zwischen der Ablauffläche 50 und der Deckelfläche 100.

Der bei eingesetztem Schutzdeckel 98 höchste Punkt desselben liegt unterhalb des höchsten Punkts der vier Pyramiden 46, so daß bei aufeinander gestapelten Sterilbehältern 10 der auf dem Deckel 14 gelagerte Behälter 12 nur auf den Spitzen 48 der Pyramiden 46 aufliegt, die Schutzdeckel 98 jedoch nicht berührt.

Trifft bei einem im wesentlichen horizontal gelagerten Sterilbehälter 10 Flüssigkeit auf die Deckeloberseite 37, beispielsweise auf den Schutzdeckel 98, so kann diese aufgrund der Wölbung des Schutzdeckels 98 zu den kurzen bzw. langen Seiten 102 bzw. 104 abfließen. Von den langen Seiten 104 gelangt die Flüssigkeit auf die Einströmkanten 40, von denen die Flüssigkeit aufgrund von deren Neigung zu den Seiten des Deckels 14 abfließen kann.

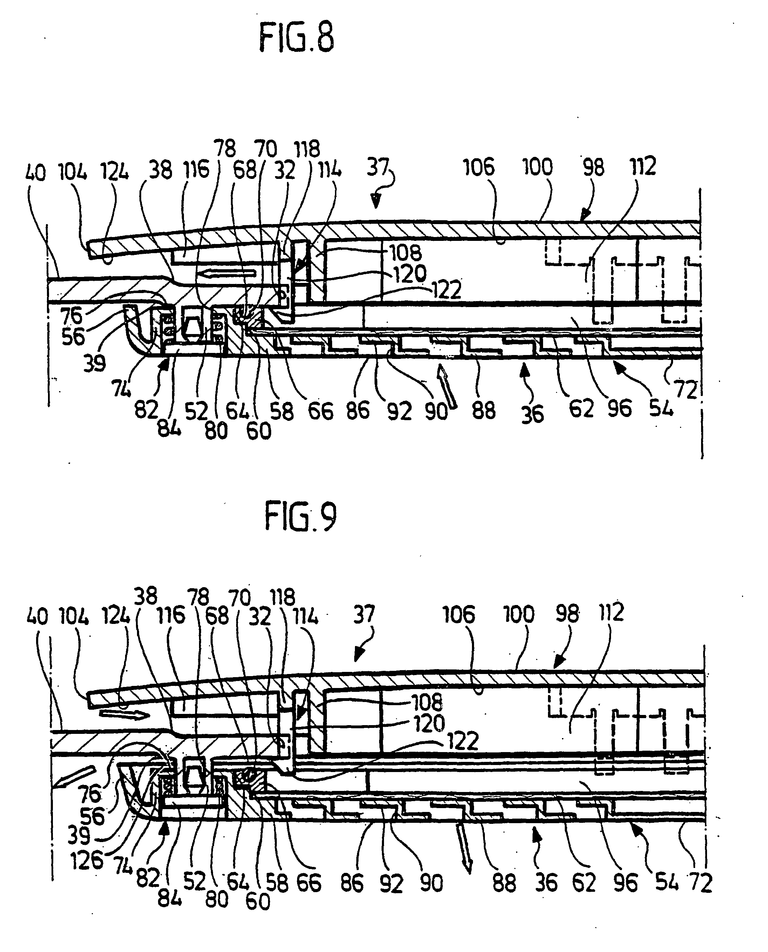

Bei aufgesetztem Schutzdeckel 98 wird eine in einer Seitenansicht im wesentlichen eine doppeltkonvex linsenförmige Einströmöffnung 124 gebildet, durch die ein gasförmiges Fluid ein- und ausströmen kann. Das Fluid strömt dann im wesentlichen parallel zur Deckelebene 15, durch die Öffnung 26 im wesentlichen senkrecht dazu. In einer in Figur 8 dargestellten Grundstellung liegt die Filterkassette 54 mittels des Dichtringes 70 abgedichtet an der Deckelunterseite 36 an. Wird der Sterilbehälter 10 mit Heißdampf beaufschlagt, dann strömt dieser wie vorgenannt beschrieben bis an den Dauerfilter 62 heran. Er tritt durch diesen durch und folgt einem durch den Trägersteg 92, die Leiste 90 und den Schutzsteg 88 gebildeten Strömungskanal ins Innere des Behälters 12.

Übersteigt ein von außen auf den Sterilbehälter 10 einwirkender Druck eine mittels den Spiralfedern 80 einstellbare Kraft, dann wirkt die federnd am Deckel 14 gelagerte Filterkassette 54 als Überdruckventil. Die Filterkassette 54 hebt von der Deckelunterseite 36 ab und öffnet einen in der Grundstellung verschlossenen Strömungskanal 126, wie in Figur 9 dargestellt. Heißdampf kann jetzt nicht nur durch das Dauerfilter 62 hindurchtreten, sondern auch über den Strömungskanal 126 ins Innere des Behälters 12 gelangen. Sobald sich der Druckgradient wieder verringert, drücken die Spiralfedern 80 die Filterkassette 54 an die Deckelunterseite zurück, so daß Ein- und Ausströmen von Dampf oder Gas nur über den Dauerfilter 62 möglich ist. In den Figuren 8 und 9 ist das Aus- bzw. Einströmen eines Gases oder Dampfes anhand der Pfeile angedeutet.

Der Behälter 12 kann wahlweise aus Metall oder Kunststoff hergestellt sein. Der Deckel 14 ist vorzugsweise vollständig aus einem Kunststoff hergestellt, beispielsweise aus Polyetheretherketon oder Polyphenylensulfon. Der Dauerfilter ist vorzugsweise aus Polytetrafluorethylen hergestellt.

## Patentansprüche

1. Sterilbehälter (10), insbesondere zur Aufnahme und sterilen Aufbewahrung von chirurgischem Besteck oder Material, umfassend einen durch einen Behälterboden und Behälterwände gebildeten Aufnahmeraum, einen Deckel (14) zum Verschließen des Aufnahmeraums und eine von einem Sterilfilter (62) verschließbare Gasaustauschöffnung (26), wobei eine das Sterilfilter (62), einen Träger (58) und ein Halteelement (66) umfassende Filtereinheit (54) vorgesehen ist, das Sterilfilter (62) zwischen dem Träger (58) und dem Halteelement (66) gehalten ist und die Filtereinheit (54) am Deckel (14) und/oder am Behälterboden gelagert ist, **dadurch gekennzeichnet, daß** die Filtereinheit in Form einer einteiligen Filterkassette (54) ausgebildet ist und daß der Träger (58), das Sterilfilter (62) und das Halteelement (66) untrennbar miteinander verbunden sind.

2. Sterilbehälter nach Anspruch 1, **dadurch gekennzeichnet, daß** der Träger (58) mindestens eine Trägerauflage (92) zum Lagern des Sterilfilters (62) und mindestens eine an die Trägerauflage (92) angrenzende Trägerdurchbrechung aufweist.

3. Sterilbehälter nach Anspruch 2, **dadurch gekennzeichnet daß** mindestens eine die mindestens eine Trägerdurchbrechung beabstandet bedeckende Trägerdurchbrechungsabdeckung (88) vorgesehen ist.

4. Sterilbehälter nach einem der voranstehenden Ansprüche, **dadurch gekennzeichnet, daß** die Filtereinheit (54) bewegbar gelagert ist, daß die Filtereinheit (54) in einer Schließstellung einen Strömungspfad (126) schließt und in einer Durchlaßstellung den Strömungspfad (126) öffnet, so daß ein Gasaustausch in der Schließstellung nur durch das Sterilfilter (62) und in der Durchlaßstellung durch das Sterilfilter (62) und/oder durch den Strömungspfad (126) ermöglicht wird.

5. Sterilbehälter nach Anspruch 4, **dadurch gekennzeichnet, daß** die Filtereinheit (54) in der Schließstellung am Sterilbehälter (10) unter Vorspannung gehalten ist.

6. Sterilbehälter nach einem der voranstehenden Ansprüche, **dadurch gekennzeichnet, daß** ein Dichtelement (70) zur gasdichten Lagerung der Filtereinheit (54) vorgesehen ist.

7. Sterilbehälter nach Anspruch 6, **dadurch gekennzeichnet, daß** das Dichtelement einen am Halteelement (66) gelagerten Dichtring (70) umfaßt.

8. Sterilbehälter nach einem der voranstehenden Ansprüche, **dadurch gekennzeichnet, daß** ein Überdruckventil (54, 52, 80) vorgesehen ist, daß das Überdruckventil (54, 52, 80) so angeordnet ist, daß es in einer Grundstellung eine Schließstellung einnimmt und daß es eine Durchlaßstellung einnimmt, wenn ein Druck in einer Umgebung des Sterilbehälters (10) einen Druck im Sterilbehälter (10) um eine vorgegebene Druckdifferenz übersteigt.

9. Sterilbehälter nach Anspruch 8, **dadurch gekennzeichnet, daß** das Überdruckventil (54, 52, 80) die Filtereinheit (54) umfaßt.

10. Sterilbehälter nach einem der voranstehenden Ansprüche, **dadurch gekennzeichnet, daß** die Filtereinheit (54) auf einer Innenseite (36) des Deckels (14) gelagert ist.

11. Sterilbehälter nach einem der voranstehenden Ansprüche, **dadurch gekennzeichnet, daß** ein die Filtereinheit (54) beabstandet bedeckendes Schutzelement (98) vorgesehen ist.

12. Sterilbehälter nach Anspruch 11, **dadurch gekennzeichnet, daß** das Schutzelement (98) auf einer Außenseite (37) des Deckels (14) angeordnet ist.

13. Sterilbehälter nach einem der Ansprüche 11 oder 12, **dadurch gekennzeichnet, daß** das Schutzelement (98) mittels einer Schnapp- oder Rastverbindung (120, 122) mit dem Deckel (14) verbindbar ist.

14. Sterilbehälter nach einem der Ansprüche 11 bis 13, **dadurch gekennzeichnet, daß** das Schutzelement (98) mit einem Innenrand (28) der Gasaustauschöffnung (26) verrastbar ist.

15. Sterilbehälter nach einem der Ansprüche 11 bis 14, **dadurch gekennzeichnet, daß** das Schutzelement (98) in Richtung auf den Sterilbehälter (10) abstehende, mit einem Rastvorsprung (122) versehene elastische Federarme (120) umfaßt.

16. Sterilbehälter nach einem der Ansprüche 11 bis 15, **dadurch gekennzeichnet, daß** zwischen dem Schutzelement (98) und dem Deckel (14) mindestens eine mit der Gasaustauschöffnung (26) in Fluidverbindung stehende Gasdurchtrittsöffnung (124) vorgesehen ist, die so angeordnet ist, daß eine Gasströmung in einer im wesentlichen quer zur Durchlaßrichtung des Sterilfilters (62) verlaufenden Strömungsrichtung ermöglicht wird.

17. Sterilbehälter nach einem der voranstehenden Ansprüche, **dadurch gekennzeichnet, daß** eine auf der Außenseite (37) des Deckels (14) angeordnete und von der Gasaustauschöffnung (26) weg weisende, nach außen relativ zu einer Deckelebene (15) abfallende Einströmkante (40) vorgesehen ist.

18. Sterilbehälter nach einem der voranstehenden Ansprüche, **dadurch gekennzeichnet, daß** der Deckel (14) mindestens ein Abstandselement (46) aufweist zum Stapein eines weiteren auf dem Sterilbehälter (10), so daß ein Gasaustausch durch die Gasaustauschöffnung (26) bei gestapelten Sterilbehältern möglich ist.

19. Sterilbehälter nach einem der voranstehenden Ansprüche, **dadurch gekennzeichnet, daß** das Sterilfilter ein Dauerfilter (62) ist, insbesondere ein aus Polytetrafluorethylen (PTFE) hergestelltes.

20. Sterilbehälter nach einem der voranstehenden Ansprüche, **dadurch gekennzeichnet, daß** der Deckel (14) aus einem Kunststoff hergestellt ist, insbesondere aus Polyetheretherketon (PEEK) oder Polyphenlylensulfon (PPSU).

## Claims

1. Sterile container (10), in particular for the holding and sterile storage of surgical instruments or material, comprising a holding space, which is defined by a container base and container walls, a lid (14) for closing the holding space and a gas exchange opening (26), which can be closed off by a sterile filter (62), wherein there is a filter unit (54), which comprises the sterile filter (62), a carrier (58) and a holding element (66), wherein the sterile filter (62) is held between the carrier (58) and the holding element (66), and wherein the filter unit (54) is mounted on the lid (14) and/or on the container base, **characterized in that** the filter unit is configured in the form of a single-part filter cartridge (54), and **in that** the carrier (58), the sterile filter (62), and the holding element (66) are nonreleasably connected to one another.

2. Sterile container according to claim 1, **characterized in that** the carrier (58) has at least one carrier support (92) for supporting the sterile filter (62), and at least one carrier aperture which adjoins the carrier support (92).

3. Sterile container according to claim 2, **characterized in that** there is at least one carrier aperture cover (88) which covers the at least one carrier aperture at a spacing therefrom.

4. Sterile container according to one of the preceding claims, **characterized in that** the filter unit (54) is mounted moveably, **in that** the filter unit (54), in a closed position, closes a flow path (126) and, in a flow-permitting position, opens the flow path (126), so that gas exchange in the closed position is possible only through the sterile filter (62) and in the flow-permitting position is possible through the sterile filter (62) and/or through the flow path (126).

5. Sterile container according to claim 4, **characterized in that** the filter unit (54), in the closed position, is held on the sterile container (10) under prestress.

6. Sterile container according to one of the preceding claims, **characterized in that** there is a sealing element (70) for mounting the filter unit (54) in a gastight manner.

7. Sterile container according to claim 6, **characterized in that** the sealing element comprises a sealing ring (70) mounted on the holding element (66).

8. Sterile container according to one of the preceding claims, **characterized in that** there is a pressure-relief valve (54, 52, 80), **in that** the pressure-relief valve (54, 52, 80) is disposed in such a way that in a basic position it adopts a closed position and that it adopts a flow-permitting position when a pressure in the vicinity of the sterile container (10) exceeds a pressure in the sterile container (10) by a predetermined pressure difference.

9. Sterile container according to claim 8, **characterized in that** the pressure-relief valve (54, 52, 80) comprises the filter unit (54).

10. Sterile container according to one of the preceding claims, **characterized in that** the filter unit (54) is mounted on an inner side (36) of the lid (14).

11. Sterile container according to one of the preceding claims, **characterized in that** there is a protective element (98) which covers the filter unit (54) at a spacing therefrom.

12. Sterile container according to claim 11, **characterized in that** the protective element (98) is disposed on an outer side (37) of the lid (14).

13. Sterile container according to either of claims 11 and 12, **characterized in that** the protective element (98) can be connected to the lid (14) by means of a snap-action or latching connection (120, 122).

14. Sterile container according to one of claims 11 to 13, **characterized in that** the protective element (98) can be latched to an inner rim (28) of the gas exchange opening (26).

15. Sterile container according to one of claims 11 to 14, **characterized in that** the protective element (98) comprises elastic spring arms (120) which protrude toward the sterile container (10) and are provided with a latching projection (122).

16. Sterile container according to one of claims 11 to 15, **characterized in that** between the protective element (98) and the lid (14) there is at least one opening for the passage of gas (124), which is in fluid communication with the gas exchange opening (26) and is disposed in such a way that gas flow is made possible in a direction of flow running substantially transversely with respect to the flow-permitting direction of the sterile filter (62).

17. Sterile container according to one of the preceding claims, **characterized in that** there is an inflow edge (40), which is disposed on the outer side (37) of the lid (14), faces away from the gas exchange opening (26) and slopes downward toward the outside relative to a lid plane (15).

18. Sterile container according to one of the preceding claims, **characterized in that** the lid (14) has at least one spacer element (46) for stacking a further sterile container on the sterile container (10), so that gas exchange through the gas exchange opening (26) is possible with stacked sterile containers.

19. Sterile container according to one of the preceding claims, **characterized in that** the sterile filter is a long-term filter (62), in particular made from polytetrafluoroethylene (PTFE).

20. Sterile container according to one of the preceding claims, **characterized in that** the lid (14) is made from a plastic, in particular from polyether ether ketone (PEEK) or polyphenylene sulfone (PPSU).

## Revendications

1. Récipient stérile (10), en particulier pour la réception et la conservation stérile d'une trousse chirurgicale ou de matériau chirurgical, comprenant un espace de réception formé par un fond de récipient et par des parois de récipient, un couvercle (14) pour fermer l'espace de réception et une ouverture d'échange de gaz (26) qui peut être fermée par un filtre stérile (62), dans lequel est prévue une unité de filtre (54) comprenant le filtre stérile (62), un support (58) et un élément de retenue (66), dans lequel le filtre stérile (62) est retenu entre le support (58) et l'élément de retenue (66), et l'unité de filtre (54) est montée sur le couvercle (14) et/ou sur le fond de récipient, **caractérisé en ce que** l'unité de filtre est réalisée sous forme d'une cassette de filtre (54) d'une seule pièce, et **en ce que** le support (58), le filtre stérile (62) et l'élément de retenue (66) sont reliés les uns aux autres de manière inséparable.

2. Récipient stérile selon la revendication 1, **caractérisé en ce que** le support (58) présente au moins une couche de support (92) pour poser le filtre stérile (62), et au moins une percée de support adjacente à la couche de support (92).

3. Récipient stérile selon la revendication 2, **caractérisé en ce qu'**il est prévu au moins un recouvrement de percée de support (88) recouvrant à distance ladite au moins une percée de support.

4. Récipient stérile selon l'une des revendications précédentes, **caractérisé en ce que** l'unité de filtre (54) est montée mobile, **en ce que** l'unité de filtre (54) ferme une voie d'écoulement (126) dans une position de fermeture et ouvre la voie d'écoulement (126) dans une position de passage, de sorte qu'un échange de gaz est possible dans la position de fermeture seulement à travers le filtre stérile (62) et dans la position de passage à travers le filtre stérile (62) et/ou à travers la voie d'écoulement (126).

5. Récipient stérile selon la revendication 4, **caractérisé en ce que** dans la position de fermeture, l'unité de filtre (54) est maintenue sous précontrainte contre le récipient stérile (10).

6. Récipient stérile selon l'une des revendications précédentes, **caractérisé en ce qu'**il est prévu un élément d'étanchéité (70) pour monter l'unité de filtre (54) de manière étanche aux gaz.

7. Récipient stérile selon la revendication 6, **caractérisé en ce que** l'élément d'étanchéité comprend un joint d'étanchéité (70) monté sur l'élément de retenue (66).

8. Récipient stérile selon l'une des revendications précédentes, **caractérisé en ce qu'**il est prévu une soupape de surpression (54, 52, 80), **en ce que** la soupape de surpression (54, 52, 80) est agencée de telle sorte qu'elle occupe une position de fermeture dans une position de base et **en ce qu'**elle occupe une position de passage lorsqu'une pression d'un environnement du récipient stérile (10) dépasse d'une différence de pression déterminée une pression régnant dans le récipient stérile (10).

9. Récipient stérile selon la revendication 8, **caractérisé en ce que** la soupape de surpression (54, 52, 80) comprend l'unité de filtre (54).

10. Récipient stérile selon l'une des revendications précédentes, **caractérisé en ce que** l'unité de filtre (54) est montée sur une face intérieure (36) du couvercle (14).

11. Récipient stérile selon l'une des revendications précédentes, **caractérisé en ce qu'**il est prévu un élément de protection (98) recouvrant à distance l'unité de filtre (54).

12. Récipient stérile selon la revendication 11, **caractérisé en ce que** l'élément de protection (98) est agencé sur une face extérieure (37) du couvercle (14).

13. Récipient stérile selon l'une ou l'autre des revendications 11 et 12, **caractérisé en ce que** l'élément de protection (98) peut être relié au couvercle (14) par une liaison à encliquetage ou à enclenchement (120, 122).

14. Récipient stérile selon l'une des revendications 11 à 13, **caractérisé en ce que** l'élément de protection (98) peut être enclenché avec le bord intérieur (28) de l'ouverture d'échange de gaz (26).

15. Récipient stérile selon l'une des revendications 11 à 14, **caractérisé en ce que** l'élément de protection (98) présente en direction du récipient stérile (10) des bras élastiques en saillie formant ressort (120) pourvus d'une saillie d'enclenchement (122).

16. Récipient stérile selon l'une des revendications 11 à 15, **caractérisé en ce qu'**il est prévu entre l'élément de protection (98) et le couvercle (14) au moins une ouverture de passage de gaz (124) en communication fluidique avec l'ouverture d'échange de gaz (26), laquelle est agencée de telle sorte qu'un écoulement de gaz est possible dans une direction d'écoulement s'étendant sensiblement transversalement à la direction de passage du filtre stérile (62).

17. Récipient stérile selon l'une des revendications précédentes, **caractérisé en ce qu'**il est prévu une arête d'écoulement (40) inclinée vers l'extérieur par rapport à un plan de couvercle (15), agencée sur la face extérieure (37) du couvercle (14) et orientée en éloignement de l'ouverture d'échange de gaz (26).

18. Récipient stérile selon l'une des revendications précédentes, **caractérisé en ce que** le couvercle (14) présente au moins un élément d'écartement (46) pour empiler un autre récipient stérile sur le récipient stérile (10), de sorte qu'un échange de gaz est possible à travers l'ouverture d'échange de gaz (26) lorsque les récipients stériles sont empilés.

19. Récipient stérile selon l'une des revendications précédentes, **caractérisé en ce que** le filtre stérile est un filtre permanent (62) fabriqué en particulier en polytétrafluoréthylène (PTFE).

20. Récipient stérile selon l'une des revendications précédentes, **caractérisé en ce que** le couvercle (14) est fabriqué en matière plastique, en particulier en polyétheréthercétone (PEEK) ou en polyphénylènesulfone (PPSU).
